(19) Europäisches Patentamt
European Patent Office
Office européen des brevets

(11) Publication number: **0 037 144**
**A2**

(12) **EUROPEAN PATENT APPLICATION**

(21) Application number: 81200316.8

(22) Date of filing: 23.03.81

(51) Int. Cl.³: A 61 K 31/28
A 61 K 37/26, A 23 K 1/16
A 23 L 1/30

(30) Priority: 31.03.80 US 136018

(43) Date of publication of application: 07.10.81 Bulletin 81 40

(84) Designated Contracting States: AT BE CH DE FR GB IT LI LU NL SE

(71) Applicant: THE PROCTER & GAMBLE COMPANY
301 East Sixth Street
Cincinnati Ohio 45201(US)

(72) Inventor: Riley, Dennis Patrick
1457 Longacre Dr.
Cincinnati, OH 45240(US)

(72) Inventor: Anderson, Mark Morgan
1534 Acreview Drive
Cincinnati, OH 45240(US)

(72) Inventor: Rotruck, John Truman
102 Peabody Drive
Oxford, OH 45056(US)

(74) Representative: Suslic, Lydia et al,
Procter & Gamble European Technical Center
Temselaan 100
B-1820 Strombeek-Bever(BE)

(54) **Chromium complexes as dietary supplements and pharmaceutical agents.**

(57) Coordinatively saturated, stable chromium (III) complexes provide dietary chromium supplementation for humans and lower animals and are useful hypoglycemic pharmaceutical agents for treating diabetes, especially when used in conjunction with insulin therapy. The chromium complexes herein are of the general type

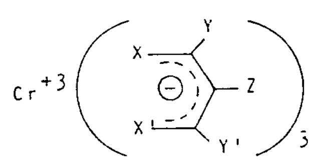

wherein the X substituents can be oxygen, sulfur or nitrogen, and the Y and Z substituents can be any of a variety of chemical moieties.

EP 0 037 144 A2

Croydon Printing Company Ltd

# CHROMIUM COMPLEXES AS DIETARY SUPPLEMENTS AND PHARMACEUTICAL AGENTS

Dennis P. Riley
Mark M. Anderson
John T. Rotruck

## Technical Field

The instant invention relates to chromium(III) complexes and their use as dietary supplements and hypoglycemic pharmaceutical agents in humans and lower animals.

Chromium has been determined to be an essential micronutrient for the maintenance of normal glucose tolerance in animals. The chromium cation exists in oxidation states of plus II, plus III and plus VI. Chromium(II) is very readily oxidized to chromium(III) on exposure to atmospheric oxygen. Chromium(VI) is toxic to man and animals. The chromium complexes used in this invention have the chromium cation in the (III) oxidation state.

Chromium(III) is safe and required in a proper dietary regimen of animals and humans. Thus, as used herein, "chromium" is intended to mean the chromium cation in the III oxidation state. The action of chromium is closely associated with that of insulin. Chromium deficiency in humans has been reported as the cause of a reduced response of insulin-sensitive tissue to the hormone, manifested by impaired glucose metabolism.

The use of various chromium(III) salts for supplementing diets of mammals has been reported in the

scientific literature. Persons particularly in need of chromium(III) supplementation include diabetics (diabetes mellitus) and those suffering from a dietary chromium deficiency.

Chromium deficiency may result in impaired glucose metabolism in one or more of the following situations: (1) dietary chromium deficiency, (2) juvenile-onset diabetes, and (3) maturity-onset diabetes.

In the case of dietary chromium deficiency, if a mammal obtains less chromium from its diet than is used or excreted from the body on a daily basis, the resulting negative chromium balance will eventually deplete body stores of chromium to the point where many bodily functions, especially insulin-sensitive processes, are impaired. Dietary supplementation with a form of chromium that is absorbable from the gastrointestinal tract and able to undergo biological interaction with insulin-sensitive systems would alleviate this situation.

In juvenile-onset diabetes, there is essentially a complete cessation of insulin production by the pancreas. Lack of circulating insulin results in severe hyperglycemia. Insulin therapy is required to maintain normal blood glucose levels, but treatment is difficult and often results in wide variations in blood glucose values. In addition, it is known that elevated blood glucose results in mobilization of body stores of chromium which is then nearly quantitatively excreted in the urine. Thus, it is apparent that a victim of juvenile-onset diabetes would mobilize and excrete more chromium than a normal individual. If this chromium is not replaced via the diet or by therapeutic supplementation, the resulting chromium deficiency may induce a refractory response to exogenous insulin, exacerbating the diabetic symptoms and requiring ever increasing doses of insulin to maintain normal blood glucose levels. A biologically-active form of chromium,

given either as a dietary supplement or as an adjunct to, or in combination with, insulin therapy, would help to prevent the development of this situation and, in some instances, allow the amount of insulin administered to maintain normal blood glucose levels to be substantially decreased.

Maturity-onset diabetes presents a different set of conditions, but with an end result similar to that of juvenile-onset diabetes. In maturity-onset diabetes, the pancreas continues to secrete insulin, often at higher than normal levels. However, due to impaired tissue sensitivity or defective insulin, this endogenous insulin elicits little or no physiological response. Normal blood glucose levels are approximated either by injection of exogenous insulin or by oral administration of hypoglycemic drugs which stimulate the pancreas to produce even more insulin. As in juvenile-onset diabetes, the abnormally high blood glucose levels would be expected to lead to increased chromium mobilization and excretion, resulting in eventual chromium deficiency and aggravation of the diabetic situation. Since chromium is necessary for optimum *in vivo* action of insulin, the importance of improving the effectiveness of insulin in a maturity-onset diabetic by administering an absorbable and effiicacious form of chromium is clear. In those diabetic individuals who are chromium deficient, this would allow lowering the total dosage of insulin or oral hypoglycemic drug, even to the point that administration of insulin or oral hypoglycemic drugs could be halted without affecting the patient.

One paradox of chromium deficiency is that while many foods naturally contain entirely adequate levels of chromium, the chromium is lost during processing. With the advent and prevalence of processed foods, it has become increasingly necessary to supplement the diet with chromium. This task has proved to be difficult because the chemical form in which chromium(III) is presented to

the body is highly determinative of its extent of absorption from the gastrointestinal tract, and thus its bioactivity, at effective, yet safe dosage levels.

It is an object of the present invention to provide chromium(III) in a stable, yet metabolically useful, form.

In accordance with this invention, certain chromium(III) complexes in safe and effective amounts can be used to treat impaired glucose metabolism due to diabetes mellitus, as well as to treat more generalized problems associated with chromium deficiency.

## Background Art

Many of the chemically familiar chromium compounds have been found to be ineffective for delivering chromium(III) ions orally to the body. Accordingly, efforts to treat mammals in need of the chromium(III) ion have involved the use of organic complexes of chromium which are somewhat ill-defined and rather unstable.

U.S. Patent 3,914,410, Godfrey (1975) discloses addition of trace amounts of certain chromium salts to sugar to produce a fortified composition which is said to reduce the severity or incidence of atherosclerosis or adult-onset diabetes. Unrefined sugar contains appreciable quantities of chromium, but as the sugar is refined the chromium is removed.

U.S. Patent 3,925,433, Abdel-Monem, et al. (1975) describes complexes formed between chromium and α-amino acids. The chromium in these complexes is said to be readily absorbed, distributed and utilized within the biochemical system of animals and humans. The rate of absorption and distribution is said to be substantially higher than that of chromium chloride.

Shwartz and Mertz, Archives of Biochemistry and Biophysics, Vol. 85, p. 293 (1959) state that very stable chromium complexes, for example the bidentate chromium acetylacetonate and several ethylenediamine complexes, seem to be metabolically inert.

Mertz, Nutrition Reviews 33, No. 5, p. 130 (May 1975) states that "simple" chromium compounds, for example the chloro, aquo, or acetate coordinate chromium compounds or complexes, do not meet the criteria of absorption and bioavailability needed for use as cofactors for insulin.

## Disclosure of Invention

EPO Patent Application N° 80200219.6 - filed March 7, 1980 - publication N° 0016496, ——————— discloses the use of chromium(III) acetylacetonate as a dietary supplement, in conjunction with insulin, and the like. The present invention employs non-acetylacetonate chromium(III) complexes of the formula A:

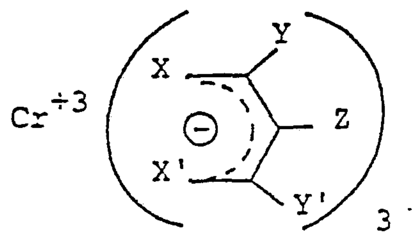

wherein the substituent groups are as follows: X and X' = O, NH, S, $NR_1$; Y and Y' = alkyl, aryl, H, $CO_2R_2$, $SR_1$, $OR_1$, perfluoroalkyl, perfluoroaryl; Z = $NO_2$, H, $CO_2R_2$, $NHCOR_1$, CN, $OR_1$, $COR_1$, SCN, 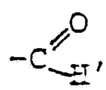, perfluoroalkyl, alkyl, aryl, perfluoroaryl, specifically excluding halogen; with $R_1$ as $C_1$-$C_{22}$ alkyl or aryl (e.g., phenyl, tolyl, xylyl, and the like) and $R_2$ as $C_1$-$C_{22}$ alkyl; groups Y and Y' can be the same or different, and X and X' can be the same or different, provided that when X and X' are both oxygen, and Y and Y' are both methyl, Z is a substituent other than hydrogen.

It is to be understood that the chromium(III) complexes herein are hexacoordinated. Accordingly, mixtures of the complexing agents can be used to form "mixed" chromium complexes of the foregoing type.

The present invention encompasses the use of the aforesaid chromium complexes as dietary supplements.

This invention also provides a convenient method for supplementing foods from which the naturally occurring chromium has been removed by means of the disclosed bioavailable, bioactive chromium complexes.

This invention also encompasses a method for treating diabetics comprising administering safe and effective amounts of the aforesaid chromium complexes.

Chromium is known to be a co-factor for insulin. Accordingly, this invention also provides a method for treating diabetic patients by concurrently administering safe and effective amounts of the chromium complexes and insulin to the diabetic.

The heat stability of the chromium complexes employed herein allows them to be incorporated in food products prepared at high temperatures without loss of nutritional value. Examples of such food products include those obtained from soybean protein.

In accordance with the foregoing, the instant invention encompasses the aforesaid chromium complexes in unit dosage forms, in combination with insulin, in combination with hypoglycemic agents, and in foods and dietary supplements, e.g., vitamin/mineral preparations.

## Best Mode

The chromium(III) complexes of the present invention are known materials, and their syntheses form no part of this invention. Appropriate syntheses of these complexes are disclosed in the literature, as described more fully hereinafter.

Of the several types of chromium(III) complexes encompassed by formula A, hereinabove, the following are preferred: compounds of formula A wherein X and X' are each oxygen, Y and Y' are each hydrogen and Z can be

H (i.e., malonaldehyde complex); compounds wherein X and X' are each O, Y is H, Y' is $CH_3$ and Z can be H (i.e., 3-oxobutanal complex) both <u>cis</u> and <u>trans</u>; compounds wherein X and X' are each O, Z is H, and Y and Y' are each phenyl; compounds wherein X is O, X' is N-phenyl or N-substituted phenyl such as p-tolyl, o-tolyl, and the like, and Y, Y' and Z are each H; and compounds wherein X and X' are each O, Y and Y' are each $CF_3$, and Z is H. Of the foregoing compounds, the malonaldehyde and 3-oxobutanal complexes (<u>cis</u> and <u>trans</u>) are most preferred.

Within the realm of sound medical judgment, the dosage of chromium complex will vary with the particular condition being treated, the severity of the condition, and the duration of treatment employed. However, single dosage amounts range from 0.15 to 100 micrograms (μg) of chromium (as complex) per kg of body weight, preferably 1 to 10 μg per kg. (Unless otherwise specified, the unit designated "μg per kg" as used herein refers to μg Cr per kg of body weight.) The higher dosages within this range are usually employed for therapeutic use in cases of severe chromium deficiency and in diabetes therapy, whereas the lower dosages are appropriate for maintaining adequate dietary chromium levels. For therapuetic use, up to 4 dosages per day can be used routinely, but this can be varied with the needs of the patient, consistent with a sound benefit:risk ratio.

Preferably, dosages ranging from about 1 μg to about 10 μg per kg are employed when the chromium complex is administered orally.

For parenteral administration (subcutaneous, intraperitoneal, intramuscular) dosages are preferably from about 0.5 to about 70 μg per kg per day. For long-term parenteral infusion (intravenous) the most highly preferred dosage range is from about 0.15 to about 15 μg per kg per day.

The chromium complexes are sparingly soluble in water. Parenteral administration can be carried out in ethanol/water mixtures.

For purposes of oral administration, the chromium complexes can conveniently be formulated as capsules, tablets or granules. Convenient unit dosage forms of the chromium complexes comprise from about 1.5 μg to about 0.07 grams of chromium as the complex and a pharmaceutical carrier. For treatment of non-human animals, the chromium complexes are preferably incorporated in animal feed, feed supplements or feed concentrates.

The preferred concentration range of the chromium complexes as described hereinabove in unit dosage forms intended for use by humans and small domesticated animals is from about 0.15 μg to about 300 μg of Cr, more preferably from 50 μg to 250 μg of Cr. A higher concentration range from about 0.3 mg of Cr to about 200 mg of Cr is the preferred unit dosage form intended for treatment of larger, non-ruminant animals, e.g., horses and the like.

Convenient compositions for oral administration of the chromium complexes can also take the form of troches, chewable tablets and foodstuffs.

The term "pharmaceutical carrier" as used herein denotes any of the usual pharmaceutical excipients, including solid or liquid fillers, diluents, tableting aids, encapsulating substances, and the like. Some examples of the substances which can serve as pharmaceutical carriers for the chromium complexes include sugars, such as lactose, glucose and sucrose; starches, such as cornstarch and potato starch; cellulose and its derivatives, such as sodium carboxymethylcellulose, ethylcellulose, cellulose acetate and powdered tragacanth; malt; gelatin; talc; stearic acids; magnesium stearate; calcium sulfate; vegetable oils, such as peanut oil, cottonseed oil, sesame oil, olive oil, corn oil and oil

BAD ORIGINAL

of theobroma; polyols, such as propylene glycol, glycerine, sorbitol, mannitol, polyethyleneglycol; agar; alginic acid; saline; and phosphate buffer solutions, as well as other non-toxic, compatible substances typically used in pharmaceutical formulations. Wetting agents and lubricants, such as sodium lauryl sulfate, as well as coloring agents, flavoring agents and preservatives can also be present. Tableting is done using conventional techniques. Gelatin capsules are another mode of administration.

The pharmaceutical carrier employed in conjunction with the chromium complexes is used at a concentration sufficient to provide a practical size to dosage relationship. Preferably, the pharmaceutical carrier comprises from about 0.1% to about 99% by weight of total composition.

The chromium complexes can be conveniently included in standard vitamin, mineral or mixed vitamin/mineral compositions to provide an excellent dietary supplement. The chemical and heat stability of the complexes assures that they will not undesirably interact with various foodstuffs, vitamins, minerals, and pharmaceutical excipients.

It will be appreciated that the present invention is useful not only in humans but also in lower animals in treating similar disease states involving the impairment of glucose metabolism and for general overall health and well-being.

Animal feed compositions to which the chromium complexes of this invention can be added generally include as foodstuffs a cellulosic roughage component such as hay, straw, plant hulls, corn cobs, and the like. Protein-containing components such as whole grains, including corn, wheat, barley, oats, rye, millet and alfalfa are typically included.

As disclosed hereinabove, the chromium complexes employed in the practice of the present invention can be prepared using art-disclosed procedures, or obvious modifications thereof. The following articles, incorporated herein by reference, describe such procedures in great detail.

The preparation of tris(1,3-propanedialato)chromium(III), i.e., the preferred malonaldehyde Cr(III) complex herein, is described in detail in INORGANIC SYNTHESES Vol. VIII, pages 141-143, 1966. The preparation of the preferred cis- and trans- 3-oxobutanal complex, specifically cis- and trans- tris(3-oxobutanalato)chromium(III) is described in the same volume of INORGANIC SYNTHESES, pages 144-148. Likewise, the preparation of tris(4-p-toluidino-3-penten-2-onato)chromium(III) appears in the same volume, pages 149-153. The synthesis of tris(1,3-diphenyl-1,3-propanedionato)chromium(III) and tris(1,1,1-trifluoro-2,4-pentanedionato)chromium(III) are reported in INORGANIC SYNTHESES, Vol. VIII, pages 135-140, 1966. Collman, et al., J. Org. Chem. 28, 1449-55(1963), report the synthesis of tris(3-acetyl-2,4-pentanediono)-chromium(III). Collman, et al., J. Am. Chem. Soc. 83, 3529-30(1961), describe the synthesis of nitro- and other chromium(III) compounds. Heath, et al., Aust. J. Chem, 1972, 25, 2547-51, describe the synthesis of chromium(III) complexes of formula A, wherein X and X' are each sulfur, i.e., tris(dithioacetylacetonato)-chromium(III). The syntheses of various compounds of formula A wherein X' is nitrogen are reported by Collman, et al., Inorganic Chemistry Vol. 1, No. 3, August 1962, pages 499-503.

The chemical and heat stability of the chromium complexes disclosed for use herein allows them to be formulated in all manner of compositions without loss of nutritional value.

Thus, the chromium complexes of formula A can be used as diet supplements in food, in unit dosage forms, as additives to other diet supplements such as vitamins and minerals, and in combination with insulin solutions and various hypoglycemic agents.

The known forms of insulin: regular, prompt, insulin zinc and crystalline-zinc, Semilente®, isophane insulin suspension (NPH insulin) and insulin zinc suspension, Lente®, globin zinc insulin, protamine zinc insulin suspension, extended insulin zinc suspension Ultralente®, are all useful in pharmaceutical mixtures with the chromium complexes of formula A for intramuscular injection.

The oral hypoglycemic agents useful with the chromium complexes in the manner of this invention are of the known commercial types. They include the sulfonylurea compounds tolbutamide, chlorpropamide, acetohexamide and tolazamide; and the bisguanide compound phenformin.

The dosage of insulin will, of course, be determined by the attending physician, according to the needs of the patient. In the present invention, from about 5 units to about 70 units of insulin will be administered per day concurrently with the chromium complex. Likewise, the oral dosage of non-insulin hypoglycemic agents can be adjusted to the needs of the individual patient, but generally ranges from about 0.5 g. to about 5 g., on a daily basis, depending, of course, on the type of hypoglycemic agent.

The following examples illustrate the practice of this invention. The scope of the invention is not intended to be limited by the examples.

## Example I

Gelatin capsules are prepared by conventional methods, comprising:

| Ingredient | µg per capsule |
|---|---|
| Tris(1,3-propanedialato)chromium(III) | 500 |
| Starch | 55500 |

The above capsule administered orally once daily substantially helps decrease glucose level in the blood of a patient weighing approximately 70 kilograms afflicted with the diabetic conditions described herein.

## Example II

Tablets are prepared by conventional methods, as follows:

| Ingredient | µg per capsule |
|---|---|
| Tris(3-oxobutanalato)chromium(III) | 1500 |
| Lactose | 40000 |
| Starch | 2500 |
| Magnesium stearate | 1000 |

When administered orally once daily the above tablet substantially decreases the glucose level in the blood of a diabetic patient weighing approximately 70 kilograms. Ingested one per day, the tablets of Example II are also useful as dietary supplements to maintain adequate Cr(III) levels in the diet of humans and lower animals.

## Example III

Meat analog compositions containing chromium complexes are prepared in the conventional manner, as follows:

| Ingredient | Parts by Weight |
|---|---|
| Extruded soy protein granules | 29.47 |
| Soy protein binder (egg white) | 5.20 |
| Solid Crisco® shortening | 15.03 |
| Tris(1,3-diphenyl-1,3-propanedionato)-chromium(III) | 0.001 |
| Coloring | 0.04 |
| Water and flavor | to 100 |

## Example IV

A multivitamin/mineral composition for human and veterinary use is formulated as follows:

| Ingredient | Amount |
|---|---|
| Vitamin A | 5,000 USP Units |
| Vitamin D | 400 USP Units |
| Thiamine (Vitamin $B_1$) | 1.5 mg. |
| Riboflavin (Vitamin $B_2$) | 1.7 mg. |
| Niacinamide | 20.0 mg. |
| Ascorbic Acid (Vitamin C) | 60.0 mg. |
| Pyridoxine (Vitamin $B_6$) | 2.0 mg. |
| Folic Acid | 0.1 mg. |
| Pentothenic Acid | 10.0 mg. |
| Cyanocobalamin (Vitamin $B_{12}$) | 0.5 mg. |
| Tris(1,3-propanedialato)chromium(III) | 20 µg. |

Example V

A chromium fortified peanut butter composition is prepared according to the following formulation:

| Ingredient | Parts by Weight |
|---|---|
| Peanut paste | 90.0 |
| Salt | 1.2 |
| Sucrose | 5.8 |
| Refined sugar | 0.5 |
| Soybean monoglyceride | 0.7 |
| Soybean oil (iodine value 2) | 0.84 |
| Soybean oil (iodine value 107) | 0.40 |
| L threonine (extracted from egg white) | 1.5 |
| N-acetyl-1-methionine | 0.5 |
| Tris(3-acetyl-2,4-pentanediono)chromium(III) | 0.002 |

Example VII

Bleached white flour is fortified with 700 μg of tris(dithioacetylacetonato)chromium(III) per kg. of flour. The flour is used for baking and any other of its usual purposes without oxidation or degradation of the chromium complex.

Example VIII

Refined sugar is fortified with 400 μg of tris(4-p-toluidine-3-penten-2-one)chromium(III) per kg. of refined sugar. Ingestion of the chromium-fortified sugar in the usual daily amounts provides substantial portions of the body's chromium requirements.

Example IX

A unit dose of an insulin-plus-chromium pharmaceutical composition comprises:

| Ingredient | Amount |
| --- | --- |
| Insulin (commercial solution) | 10 units |
| Tris(1,3-propanedialato)chromium(III) | 70 µg |

The composition of Example IX is prepared by mixing the indicated ingredients. Daily intramuscular administration of the composition (hypodermic syringe) to a diabetic patient normalizes blood glucose levels.

Example X

An oral hypoglycemic agent in unit dosage capsule form comprises:

| Ingredient | Amount |
| --- | --- |
| Chlorpropamide | 500 mg |
| Tris(3-oxobutanalato)chromium(III) | 150 µg |

Oral administration of one gelatin capsule per day of the composition of Example X to a patient suffering from diabetes mellitus suffices to normalize blood sugar levels.

Example XI

In the composition of Example X, the chlorpropamide is replaced by a safe and effective dose of tolbutamide (5 g/day), acetohexamide (15 g/day) and tolazamide (1.5 g/day), respectively, and excellent oral hypoglycemic agents are secured in each instance.

Example XII

To a diabetic patient, a chromium complex capsule of Example I or II is orally administered before or after the intramuscular administration of insulin (commercial solution) to aid in the normalization of the blood glucose level of the patient.

Example XIII

Timothy hay is fortified with a nutritionally supplemental amount (5 g/2200 kg) of tris(1,1,1-trifluoro-2,4-pentanedionato)chromium(III) and is suitable for feeding non-ruminant animals.

23.03.81
0037144

CLAIMS

1. A composition of matter for oral administration in unit dosage form characterized by a safe and effective amount of :

A. A chromium complex of the formula:

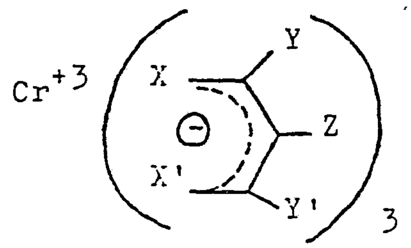

wherein: X and X' can be the same or different and are selected from O, NH, S, $NR_1$; Y and Y' can be the same or different and are selected from alkyl, aryl, H, $CO_2R_2$, $SR_1$, $OR_1$, perfluoroalkyl, perfluoroaryl, Z is selected from $NO_2$, H, $CO_2R_2$, $NHCOR_1$, CN, $OR_1$, $COR_1$, SCN, 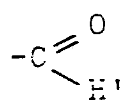, perfluoroalkyl, alkyl, aryl, perfluoroaryl, wherein $R_1$ is selected from $C_1$-$C_{22}$ alkyl or aryl and $R_2$ is selected from $C_1$-$C_{22}$ alkyl; and mixtures thereof, provided that when X and X' are both oxygen, and Y and Y' are both methyl, Z is a substituent other than hydrogen; and

B. The balance of the composition comprising a pharmaceutically-acceptable carrier.

2. A composition according to Claim 1 in unit dosage form for oral administration, characterized by from about 1.5 ug to about 0.07 grams of chromium as a complex selected from the group consisting of tris (1,3-propanedialato)-chromium (III); *cis*- and *trans*- tris (3-oxobutanalato)-chromium (III); tris (4-p-toluidino-3-penten-2-onato)-chromium (III); tris (1,3-diphenyl-1,3-propanedionate)-chromium (III); tris (1,1,1-trifluoro-2,4-pentanedionato)-

chromium (III); tris (3-acetyl-2,4-pentanediono) chromium (III); tris (dithioacetylacetonato) chromium (III), and mixtures thereof; and a pharmaceutically-acceptable carrier.

3. A food or beverage composition for human or animal use characterized by a safe and effective amount of a chromium (III) complex according to Claim 1.

4. A composition according to Claim 3, characterized by the chromium complex being selected from : tris (1,3-propanedialato) chromium (III); <u>cis</u>- and <u>trans</u>-tris (3-oxobutanalato) chromium (III); tris (4-p-toluidino-3-penten-2-onato) chromium (III); tris (1,3-disphenyl-1,3-propanedionato) chromium (III); tris (1,1,1-trifluoro-2,4-pentanedionato) chromium (III); tris (3-acetyl-2,4-pentanediono) chromium (III); tris (dithioacetylacetonato) chromium (III), and mixtures thereof.

5. A composition characterized by :
(a) a safe and effective amount of a chromium (III) complex of the formula :

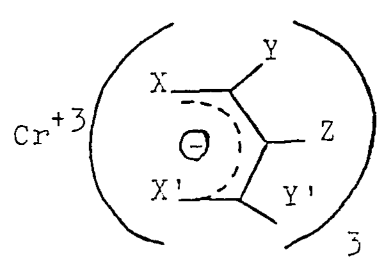

wherein: X and X' can be the same or different and are selected from O, NH, S, $NR_1$; Y and Y' can be the same or different and are selected from alkyl, aryl, H, $CO_2R_2$, $SR_1$, $OR_1$, perfluoroalkyl, perfluoroaryl; Z is selected from $NO_2$, H, $CO_2R_2$, $NHCOR_1$, CN, $OR_1$, $COR_1$,

SCN, 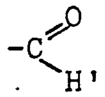 perfluoroalkyl, alkyl, aryl, perfluoroaryl, wherein $R_1$ is selected from $C_1$-$C_{22}$ alkyl or aryl and $R_2$ is selected from $C_1$-$C_{22}$ alkyl; and mixtures thereof, provided that when X and X' are both oxygen, and Y and Y' are both methyl, Z is a substituent other than hydrogen; and

(b) a safe and effective amount of insulin.

6. A composition according to Claim 5 characterized by chromium complex being selected from : tris (1,3-propanedialato) chromium (III); *cis*- and *trans*-tris (3-oxobutanalato) chromium (III); tris (4-p-toluidino-3-penten-2-onato) chromium (III); tris (1,3-diphenyl-1,3-propanedionato) chromium (III); tris (1,1,1-trifluoro-2,4-pentanedionato) chromium (III); tris (3-acetyl-2,4-pentanedione) chromium (III); tris (dithioacetylacetonato) chromium (III), and mixtures thereof.

7. A composition characterized by :

(a) a safe and effective amount of a chromium (III) complex of the formula

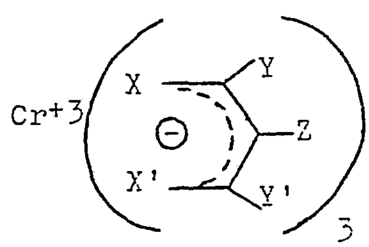

wherein: X and X' can be the same or different and are selected from O, NH, S, $NR_1$; Y and Y' can be the same or different and are selected from alkyl, aryl, H, $CO_2R_2$, $SR_1$, $OR_1$, perfluoroalkyl, perfluoroaryl, Z is selected from $NO_2$, H, $CO_2R_2$, $NHCOR_2$, CN, $OR_1$, $COR_1$,

SCN, 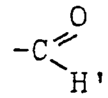 perfluoroalkyl, alkyl, aryl, perfluoroaryl, wherein $R_1$ is selected from $C_1$-$C_{22}$ alkyl or aryl and $R_2$ is selected from $C_1$-$C_{22}$ alkyl; and mixtures thereof, provided that when X and X' are both oxygen, and Y and Y' are both methyl, Z is a substituent other than hydrogen; and

(b) A safe and effective amount of hypoglycemic agent.

8. A composition according to Claim 7 characterized by said hypoglycemic agent being selected from the group consisting of sulfonylurea and bisguanide oral hypoglycemic agents.

9. A composition according to Claim 8 characterized by said chromium complex being selected from : tris (1,3-propanedialato) chromium (III); cis- and trans-tris (3-oxobutanalato) chromium (III); tris (4-p-toluidino-3-penten-2-onato) chromium (III); tris (1,3-diphenyl-1,3-propanedionato) chromium (III); tris (1,1,1-trifluoro-2,4-pentanedionato) chromium (III); tris (3-acetyl-2,4-pentanedione) chromium (III); tris (dithioacetylacetonato) chromium (III), and mixtures thereof.

10. Substance for providing to a human or lower animal in need of such treatment a nutritionally supplemental amount of a chromium (III), characterized by the formula:

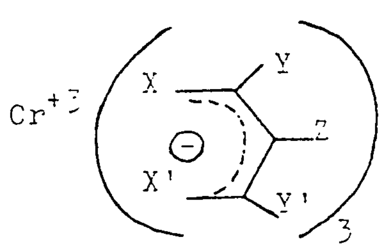

wherein: X and X' can be the same or different and are selected from O, NH, S, $NR_1$; Y and Y' can be the same or different and are selected from alkyl, aryl, H, $CO_2R_2$, $SR_1$, $OR_1$, perfluoroalkyl, perfluoroaryl; Z is selected from $NO_2$, H, $CO_2R_2$, $NHCOR_1$, CN, $OR_1$, $COR_1$, SCN, 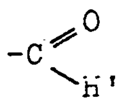 perfluoroalkyl, alkyl, aryl, perfluoroaryl, wherein $R_1$ is selected from $C_1$-$C_{22}$ alkyl or aryl and $R_2$ is selected from $C_1$-$C_{22}$ alkyl; and mixtures thereof, provided that when X and X' are both oxygen, and Y and Y' are both methyl, Z is a substituent other than hydrogen.